# EUROPEAN PATENT SPECIFICATION

(11) **EP 2 155 136 B1**
(45) Date of publication and mention of the grant of the patent: **13.06.2018**
(21) Application number: 08733717.6
(22) Date of filing: 04.04.2008
(51) Int. Cl.: A61G 13/12, A61B 5/055, A61B 6/04, A61G 13/08, G01R 33/36, A61B 5/08

(54) **PATIENT TRANSPORTER FOR IMAGING AND INTERVENTION**
PATIENTENTRANSPORTER ZUR BILDGEBUNG UND INTERVENTION
DISPOSITIF DE TRANSPORT DE PATIENT POUR L'IMAGERIE ET LES INTERVENTIONS CHIRURGICALES

(30) Priority: 06.04.2007 US 922293 P
(43) Date of publication of application: 24.02.2010
(73) Proprietor: INVIVO CORPORATION, Andover, MA 01810 (US)
(72) Inventor: PIRON, Cameron Anthony, Toronto, Ontario M4V 2H4 (CA); LUGINBUHL, Christopher Alexander, Toronto, Ontario M4L 3A5 (CA); MOHAMMADI, Hamid, M6P 3X7 (CA)
(74) Representative: de Haan, Poul Erik
(86) International application number: PCT/CA2008/000636
(87) International publication number: WO 2008/122117

(56) References cited:
- US-A- 3 814 414
- US-A- 4 131 802
- US-A- 4 379 450
- US-A- 4 989 848
- US-A- 5 799 059
- US-A1- 2004 103 477
- US-A1- 2006 241 408
- US-A1- 2007 039 101
- US-B1- 6 295 671
- US-B1- 6 459 923

## Description

### TECHNICAL FIELD

The invention relates to medical imaging systems for viewing anatomical structures and functions of a patient, such as Magnetic Resonance Imaging (MRI) and x-ray Computed Tomography (CT) imaging scanners. More particularly, this invention relates to a patient transporter which enables hospital or medical center personnel to transport a patient from an imaging scanner to another imaging scanner or to radiation therapy equipment or to a location remote from the scanner for performing additional imaging examinations or interventional procedures without the necessity for transferring the patient from one patient support device to another.

### BACKGROUND

For many clinical indications, improvements in diagnostic imaging technology have resulted in a trend away from open surgical procedures to less invasive image-guided interventional procedures. Non-invasive imaging modalities may be used to localize pathology and to navigate the anatomy for performing biopsy procedures or therapeutic interventions in lieu of more invasive surgical procedures. MRI and CT imaging provide excellent visualization of a patient's anatomy, whereas Positron Emission Tomography (PET), nuclear imaging, ultrasound, optical tomography and other imaging modalities may be used to enhance sensitivity to disease.

While it is preferable to perform interventional procedures with direct real-time image guidance, access to a patient for performing interventional procedures while the patient is positioned for imaging in a conventional MRI, CT or PET scanner is limited. These scanners require positioning of the patient inside a circular tunnel during imaging, limiting a physician's access to the patient's anatomy while images are being acquired. Open MRI scanner designs have been commercialized for interventional use; however, the magnetic field strength of these open magnets is necessarily decreased compared to conventional MR imaging scanners resulting in significant degradation of image quality. In addition, performing procedures in this setting requires the use of specialized non-magnetic interventional instruments which are expensive and suffer from decreased rigidity and sharpness compared to their magnetic equivalents. Robotic solutions have also been proposed for performing interventions with a patient positioned in a conventional imaging scanner. These solutions are expensive, and may not be appropriate for all clinical applications due to the increased patient anxiety that typically results from the lack of human contact and the lack of direct surveillance by an anesthesiologist or other medical professionals during a procedure.

Solutions have been presented for combining additional imaging modalities with MRI and/or CT with the patient positioned in the bore of the imaging scanner. For example, optical imaging technologies have been designed to operate in the bore of a MRI scanner. These solutions are limited by the requirement for using non-magnetic materials for MRI scanners, and the inability of many of these ancillary imaging modalities to be operated in high magnetic field environments. Ultrasound examinations share similar requirements as interventional procedures for accessing the patient's anatomy directly by a medical professional during scanning.

Systems have been proposed for imaging a patient using multiple imaging modalities and/or performing interventional procedures by positioning the patient on a specialized apparatus that retains the patient in the same position for multiple exams. For example, a system for transporting a patient between a CT scanner and a PET scanner has been described, in which the CT and PET scanners are aligned along a common axis with a space between them for performing interventions (US 7,254,438). This system requires permanent alignment of the CT and PET scanners end-to-end in a single room, complicating siting requirements and limiting usefulness of the scanners for single modality procedures. Systems of multiple imaging scanners and an interventional/surgical suite in which the relative positions of the imaging scanners are fixed are also known in the art. For these systems, the patient is typically constrained to move between the imaging and interventional stations using a rail-guided or other similar transporter. The patient tables proposed for use with these systems are simply flat tables, of the kind typically used for routine clinical imaging on a conventional CT or PET scanner.

Recently, a method and system (U.S. Patent US 2005-0080333) has been proposed for transporting a patient between a conventional MR, CT or PET scanner and locations away from the scanner for examinations using other ancillary imaging devices and/or for performing interventional procedures while maintaining the position of an anatomy of interest in the same position throughout. The system comprises a mobile patient transporter and a dedicated tabletop that is specially configured to support a patient comfortably with a specific anatomy of interest immobilized throughout the imaging and/or interventional procedures. Apertures in the patient support structure under the anatomy of interest allow access for interventional procedures without repositioning the patient after an imaging procedure. This allows the images obtained by the conventional imaging scanner to be used to guide the ancillary imaging exams or interventional procedures without the need for real-time MRI or CT imaging throughout. U.S. Patent US 2007-0039101 similarly teaches the use of a patient transporter that can interchangeably receive a number of dedicated tabletops, each of which is designed specifically for imaging and interventional medical procedures for a selected, specific anatomy.

A single generic patient transporter that may be configured for imaging and intervention in a particular anatomy of interest by assembling a modular patient table from components selected from a kit of table modules specialized for holding different anatomies of interest during multiple imaging or interventional procedures, is desirable to facilitate the use of 3D imaging modalities such as MRI, CT and PET to guide interventional procedures or imaging with other modalities such as ultrasound or conventional x-ray. The present invention addresses these issues.An apparatus for medical imaging and image-guided intervention according to the invention is described in claim 1. Preferred embodiments of the invention are described in the subclaims.

### FIGURES

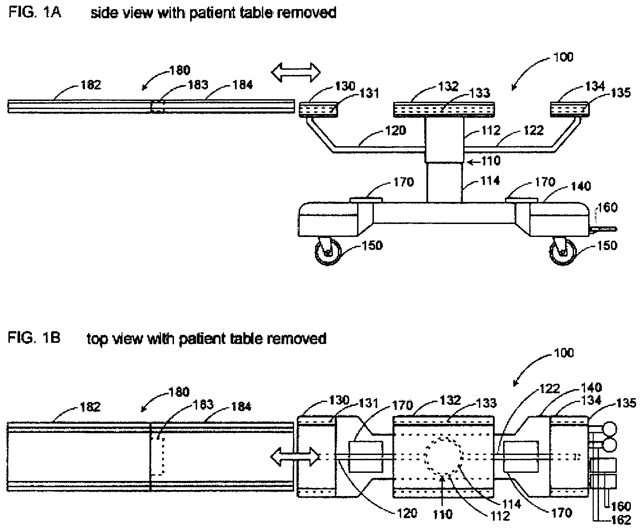

Embodiments are illustrated by way of example and not limitation in the figures of the accompanying drawings, in which like reference numerals indicate corresponding, analogous or similar elements, and in which: FIG. 1A shows a side view of a patient transporter with a modular patient table configured for routine MR imaging in accordance with an embodiment of the invention, with the patient table removed;
FIG. 1B shows a top view of the patient transporter of FIG. 1 A;
FIG. 1C shows a side view of a patient transporter with the modular patient table aligned on the transporter;
FIG. 1D is a front view of the patient transporter of FIG. 1C; FIG. 1E is a top view of the patient transporter of FIG. 1C;
FIG. 1F is a side view of the patient transporter of FIG. !C with the upper surface of the transporter lowered; FIG. 1G is a front view of the patient transporter of FIG. 1 F;
FIG. 2A shows a top view of a patient transporter with a patient table configured for prostate imaging and interventional procedures;
FIG. 2B is a top view of the patient transporter and patient table of FIG. 2A with stirrups provided on the tabletop; FIG. 2C is a top view of the patient transporter of FIG. 2C with a patient positioned on the table;
FIG. 2D is a side view of the patient transporter of FIG. 2D is a side view of the patient transporter of FIG. 2C;
FIG. 3A is a top view of an alternate exemplary patient table embodiment for prostate imaging and intervention;
FIG. 3B is a view of the patient table of FIG. 3A with a patient supported on the table;
FIG. 3C is a side view of the table of FIG. 3B;
FIG. 4A is a side view of a patient transporter including a table top for breast imaging;
FIG. 4B is a side view of a patient transporter including a table top for lung imaging;
FIG. 4C is a side view of a patient transporter including a table top for liver imaging;
FIG. 4D is a side view of a patient transporter including a table top for head imaging;
FIG 4E is a side view of the patient transporter of FIG. 4D with the RF coil removed;
FIG 4F is a side view of the patient transporter of FIG. 4D, illustrating the head support within the RF coil;
FIG. 4G is a front view of the patient transporter of FIG. 4D;
FIG. 5A shows an example of a backbone structure embodiment for a patient table compatible with an MRI scanner comprising electrical cabling and electrical connectors;
FIG. 5B shows the backbone structure of FIG. 5A with a simple patient table top attached;
FIG. 5C shows an embodiment according to the invention of a backbone structure embodiment for a patient table compatible with an MRI scanner comprising electrical cabling and electrical connectors;
FIG. 5D shows an example of a backbone structure embodiment for a patient table compatible with an MRI scanner comprising electrical cabling and electrical connectors;
FIGs. 6A - 6K illustrate various embodiments of patient transporters, each of which include gaps or spaces to provide access to an anatomy of interest of a patient positioned on the transporter; and
FIGs. 7A - 7H shows a series of exemplary patient transporter embodiments including reinforcing support structures for supporting the patient table during translation of the patient table.

It will be appreciated that for simplicity and clarity of illustration, elements shown in the figures have not necessarily been drawn to scale. For example, the dimensions of some of the elements may be exaggerated relative to other elements for clarity.

### DETAILED DESCRIPTION

In the following detailed description, numerous specific details are set forth in order to provide a thorough understanding of embodiments. However it will be understood by those of ordinary skill in the art that the embodiments may be practiced without these specific details. In other instances, well-known methods, procedures, components and circuits have not been described in detail so as not to obscure the embodiments.

A mobile patient transporter that engages with and supports a modular patient table is presented that enables hospital or medical center personnel to transport a patient from a first imaging scanner to a location remote from the first scanner for performing additional imaging examinations or interventional procedures without the necessity for transferring the patient from one patient support device to another or repositioning an anatomy of interest. The patient transporter comprises vertical supports for the patient table. The vertical supports are constructed such that open areas between the supports provide access to the patient at multiple anatomical locations from below the patient table large enough for performing ancillary imaging exams such as ultrasound imaging, or for performing medical interventions. The modular patient table may be configured using a standard patient support module, and a selection of anatomically-specific patient support modules designed to support and constrain a single selected anatomy throughout the imaging and other procedures, each of which include an interventional gap sized to allow access to the particular anatomy at least from below the patient for performing interventions in that anatomy. For a given procedure, a specialized anatomical patient support module can be selectively coupled to the standard patient support module to provide a tabletop specifically for positioning the patient for imaging of the selected anatomy.

FIGs. 1A -1F show an exemplary patient transporter with an exemplary modular patient table configured for routine MR imaging in accordance with an embodiment of the invention. FIG. 1A is a side view of a patient transporter 100 with a detachable patient table 180 that has been removed from the patient transporter 100 by a horizontal translation. FIG. 1B is a top view of the patient transporter 100 with the patient table 180 removed. The patient transporter 100 comprises a base 140 with wheels 150, a central vertical pedestal 110 rising from the center of the base 140, and two arms 120, 122 projecting horizontally from the central pedestal 110 toward the back and front ends of the patient transporter 100 respectively. The arms may have circular cross-section to enhance mechanical strength. Wheel locks (not shown) may be provided for parking the patient transporter 100. The central pedestal 110 supports a central horizontal platform 132. The arms 120, 122 angle upward near the ends of the patient transporter 100 to provide cantilevered support for additional horizontal platforms 130, 134. The arms 120, 122 may preferably be removable from the central pedestal 110, to provide increased access to a patient's anatomy of interest with the patient bed cantilevered from the pedestal 110 for performing ancillary imaging procedures, for example, an ultrasound examination, or for interventional procedures. The horizontal platforms 130, 132, 134 comprise engaging tracks 131, 133, 135 for the patient table 180 at their lateral edges. The patient table 180 may slide horizontally onto the patient transporter 100 when engaged in the engaging tracks 131, 133, 135. The engaging tracks 131, 133, 135 control alignment of the patient table 180 during translation and may preferably be configured to prevent tipping of the patient table 180 during translation. Foot-operated pedals 160 may be used to dock the patient transporter 100 to a MRI scanner (not shown) or other imaging scanner or station (not shown) or to an interventional station (not shown), as is known in the art. Additional foot-operated pedals 162 may be used to raise/lower the central pedestal 110. The central pedestal 110 may comprise telescoping segments 112, 114 arranged about a common vertical axis to allow raising/lowering of the horizontal platforms 130, 132, 134 synchronistically. Mirrors 170 may be located on the base 140 under the open spaces between the horizontal platforms 130, 132, 134 for use during ancillary imaging and/or interventional procedures. The central pedestal 110 is preferably made from materials with sufficient mechanical strength to support a patient's weight on the patient table 180 without the requirement for a large cross-sectional area of the central pedestal 110 in a horizontal plane. Preferably, the cross-sectional area of the central pedestal 110 is made as small as possible in order to increase the amount of space available adjacent to the central pedestal 110 above the base 140 to allow access to the patient through the openings between horizontal platforms 130, 132 and 134. Likewise, the cross-sectional area of the arms 120, 122 is also reduced to decrease restrictions to access to the patient. Although the patient transporter is described as including one or more horizontal platform 130, 132, and 134, it will be apparent that the central pedestal 110 and other surfaces can be sized and dimensioned to receive or support a patient tabletop, and that the horizontal platforms are not necessary.

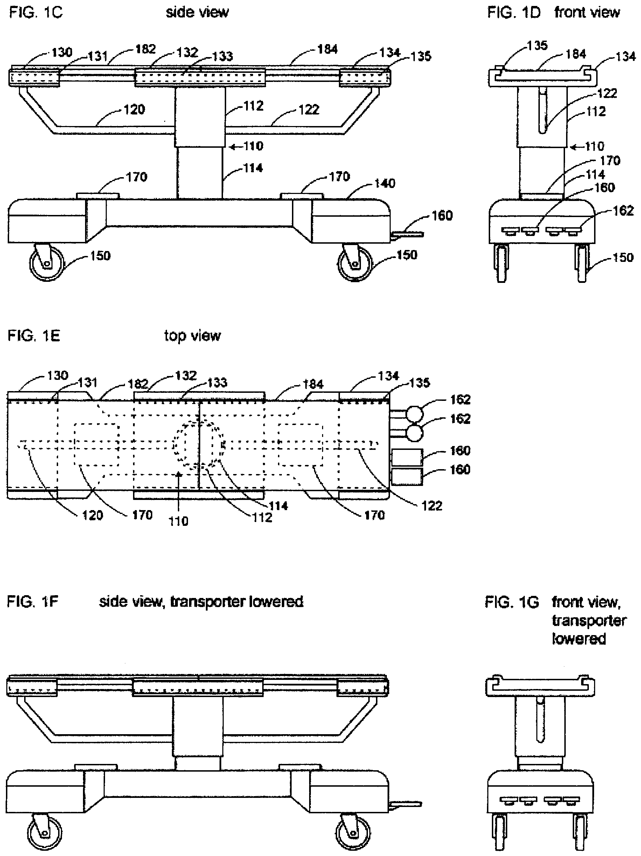

To provide a substantially flat, general purpose tabletop, the patient table 180 may comprise two segments, a substantially flat patient support module 182, and a substantially horizontal patient support module 184, joined together using a coupling element that can be a friction joint (not shown), a butt joint with biscuit 183 or dowel reinforcement (not shown), clamping devices (not shown), or any other mechanical joining method known in the art that provides sufficient longitudinal rigidity of the patient table 180 to support a patient. FIG. 1C is a side view of the patient transporter 100 with the patient table 180 supported by the horizontal support members 130, 132, 134 and engaged laterally by the lateral engaging tracks 131, 133, 135. The patient table 180 is shown centered over the patient transporter 100 in its "home" position. The patient transporter 100 may be configured such that the docking/undocking pedals 160 may only be used to dock or undock to an imaging scanner or interventional station when the patient table 180 is in the home position. In addition, wheel locks (not shown) may be configured to allow movement of the patient transporter 100 only when the patient table 180 is in the home position. FIG. 1D is a front view of the patient transporter 100 and patient table 180. FIG. 1E is a top view of the patient transporter 100 with the patient table 180 in the home position. FIG. 1F is a side view and FIG. 1G is a front view of the patient transporter 100 with the patient table 180 in the home position and the central pedestal 110 arranged in its lowest position for patient loading/unloading. While the exemplary patient transporter shown in FIG. 1 is free-moving with wheels, alternative embodiments may include patient transporters that engage with tracks on a floor, or are otherwise constrained to move along a particular path. While the exemplary patient transporter embodiment shown in FIG. 1 uses a telescoping method for providing vertical adjustment of the patient table, other standard techniques may be alternatively used, for example, ball screws, worm gears, hydraulic lifts, rack and pinions, scissor lifts, etc.

The horizontal platforms 130, 134 are shown in FIG. 1 at the same height as the central horizontal platform 132, supporting the patient bed 180 in a level horizontal position. Alternatively, a patient bed may have a more complicated geometry configured for supporting the patient in other positions, and the horizontal platforms 130, 132, 134 may be adjusted to different heights as appropriate to support the desired patient position. The arms 120, 122, may be height adjustable to allow the horizontal platforms 130, 132, 134 to be positioned at different selected heights.

The patient table 180 is preferably constructed from materials that are MRI-compatible (i.e., that are non-magnetic and non-conductive), for use with MR imaging. Examples of such materials are polycarbonate, Kevlar composite, fiberglass, interrupted carbon-fiber, wood, and other polymer or composite blends. More preferably, the patient table 180 is constructed of materials that are MRI-compatible and radiolucent. Alternatively, materials whose x-ray attenuation may be well-characterized and corrected for in CT imaging reconstructions may be used. Patient table 180 is shown assembled as a flat patient bed comprising two joined patient structural modules 182 and 184, similar to the flat patient beds that are used conventionally with MRI and CT imaging scanners. Wheels (not shown) may be incorporated into the underside of the patient table 180 to facilitate translation of the patient table 180 on the patient transporter 100. The engaging tracks 131, 133, 135, may comprise dovetail constraints or the like to prevent the patient table 180 from tipping during translation.

As described above, the patient support module 182 is a common component that is configured to be mated mechanically to a specialized table component configured to support a particular anatomy of interest for imaging and intervention. To provide specialized tabletops, the substantially horizontal patient support module 184 may be removed and replaced by specialized anatomical patient support modules (not shown in FIG. 1) that provide interventional gaps under a particular anatomy of interest that align with the spaces between horizontal support members 132, 134 when the table is in the home position. These interventional gaps provide access to a patient's anatomy of interest from below the patient table for ancillary imaging and/or interventional procedures. These specialized anatomical patient support modules may also include support structures on their upper surfaces for supporting and immobilizing a particular anatomy of interest. Patient support module 182 may preferably comprise electrical connection components (not shown in FIG. 1) for making an electrical connection between a MRI scanner and RF coil components that may be placed on or coupled to the patient table 180, more particularly to the specialized table segment. The specialized anatomical patient support modules may also comprise electrical connections to connect to table segment 182 and to RF coil components. The patient support module 182 or the specialized anatomical patient support module may also comprise integrated RF coil elements for transmitting and/or receiving RF signals from the patient's anatomy of interest during MRI scanning. Alternatively, the patient support module 182 or the specialized anatomical patient support module may comprise a receptacle for an x-ray film cartridge, or components for optical imaging, or for treatment modalities such as focused ultrasound tissue ablation.

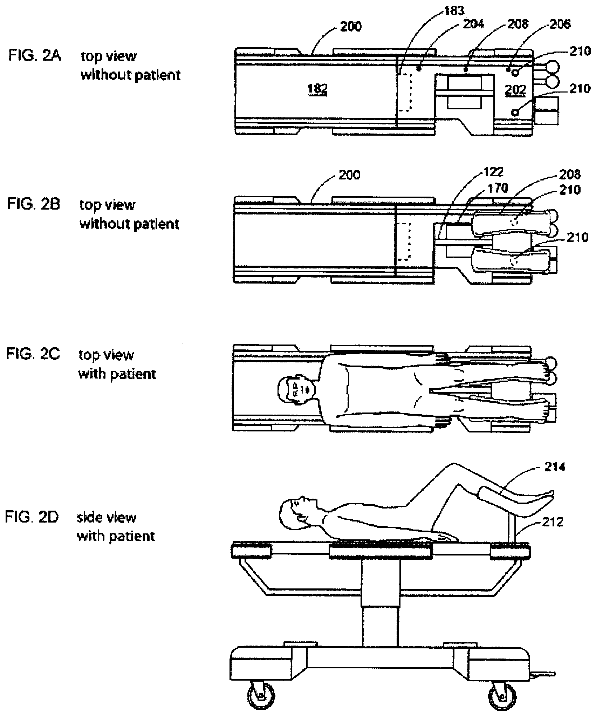

FIGs. 2A - 2D show the patient transporter 100 from FIG. 1 with patient table 180 replaced by a patient table 200 that is configured for prostate imaging and intervention. FIG. 2A is a top view of the patient transporter 100 with the prostate table 200 in the home position. The prostate table 200 is shown assembled from patient support module 182 and a specialized anatomical patient support module 202 designed for prostate imaging and intervention. Specialized anatomical patient support module 202 comprises a superior segment 204 and an inferior segment 206 joined on a lateral edge by a bridge segment 208. The gap between the superior segment 204 and the inferior segment 206 is preferably more than 12" and less than 30" in the superior-inferior direction to provide space for a physician to access a patient's perineum and rectum. The inferior segment 206 comprises receptacles 210 for vertical stirrups posts 212 (better shown in FIG. 2D). FIG. 2B is a top view showing stirrups 214 mounted on the vertical stirrups posts 212. FIG. 2C shows a patient positioned for prostate imaging and intervention on the patient transporter system of FIG. 2A. The patient's buttocks are shown positioned at the superior edge of the opening in the prostate table 200. The patient is shown lying supine with knees raised to increase access to the rectal and perineal areas from below the legs for performing interventional procedures such as prostate biopsy or brachytherapy for example, or for performing a transrectal ultrasound exam using an endorectal probe. An exemplary use of the patient transporter 100 would be to constrain the patient to be in the same positioning for a prostate MRI exam and a transrectal ultrasound-guided biopsy. The bridge segment 208 may be positioned either on the left or right lateral edge of the prostate table 200 according to the preference of the physician. The patient transporter 100 may preferably include a mirror situated in or near the medial plane in a horizontal plane near (within 12" of) the patient's prostate, allowing the physician to visualize the region of the rectum/perineum for the purpose of patient positioning, device positioning and device delivery. The prostate table 200 may additionally include shoulder braces (not shown) that may be adjusted to rest against the patient's shoulders, preventing him from sliding towards the head end of the table by pushing with his legs. FIG. 2D shows a side view of the patient transporter 100 and the prostate table 200 with a patient positioned for prostate imaging and intervention. Stirrups 214 are shown supported on vertical stirrups posts 212 that are held in the receptacles 210. The stirrups 214 may be supported on the vertical stirrups posts 212 in a fixed arrangement, or they may be attached using lockable ball joints or other such arrangements to allow adjustment of the stirrups 214 position for individual patients. While stirrups 214 that comprise leg support are shown in FIG. 2, it would be apparent to those skilled in the art that other means for supporting a patient's feet and/or legs may be substituted for stirrups, including but not limited to: heel pads, boots, or calf supports. The common table segment 182 and the specialized table segment 202 preferably comprise electrical connections for providing an RF connection between an MRI scanner and RF coil elements that may be plugged into the specialized table segment 202. For example, it may be desirable to perform a prostate MRI exam using an endorectal RF coil (not shown). The endorectal RF coil may preferably be plugged into the specialized table segment 202 (instead of into the MRI scanner directly), thereby limiting the length of external cabling required. This is desirable to reduce movement of the cabling (and potentially, the RF coil) due to the patient's motions, and to reduce the possibility of looping of the cabling. Alternatively, the endorectal RF coil may be plugged directly into the patient support module 182. Additional RF coil elements compatible with the endorectal RF coil, for example an RF coil element array that is positioned on the patient's anterior lower torso, may also be plugged into the specialized anatomical patient support module 202, thereby connecting to the MRI scanner. RF coil elements may be built into the specialized anatomical patient support module 202 to provide additional posterior coverage of the pelvis for MRI scanning. Specialized anatomical patient support module 202 may additional comprise mounting structures (not shown) for RF coil elements, patient immobilization or interventional instruments.

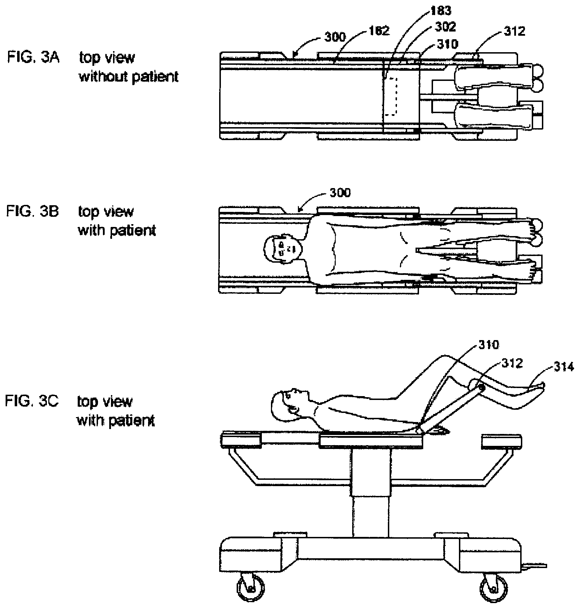

FIG. 3 shows an alternate embodiment for a patient table configuration for prostate imaging and intervention. FIG. 3A shows a top view of a prostate table 300 assembled from the patient support module 182 and a specialized prostate patient support module 302 designed for prostate imaging and intervention. The prostate specialized imaging module 302 extends inferiorly only as far as is necessary to support the patient's buttocks. The specialized prostate patient support module 302 comprises slots 310 positioned laterally for stirrups mounts 312. FIG. 3B is a top view of the patient transporter 100 and prostate table 300 with a patient positioned for prostate imaging and/or intervention. FIG. 3C shows a side view of the patient transporter system of FIG. 3A with a patient positioned on prostate table 300. The patient is shown with legs supported by stirrups 314. The stirrups 314 may be coupled to stirrups mounts 312 in a fixed arrangement, or the stirrups 314 may be attached to stirrups mounts 312 using lockable ball joints or other such arrangements to allow adjustment of the stirrups position for individual patients. The stirrup mounts 312 may be attached to the specialized prostate anatomical support module 302 by sliding into the slots 310 and locking. The embodiments shown in FIG. 2 and FIG.3 may alternatively be used for performing any other pelvic procedures for either male or female patients, including for performing a biopsy of a solid mass in the pelvis, or for performing minimally invasive therapies for cervical, ovarian or rectal cancers etc.

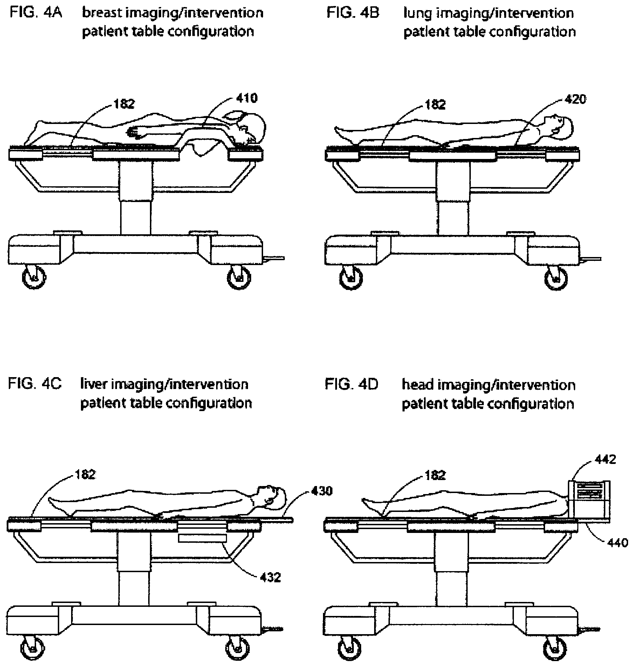

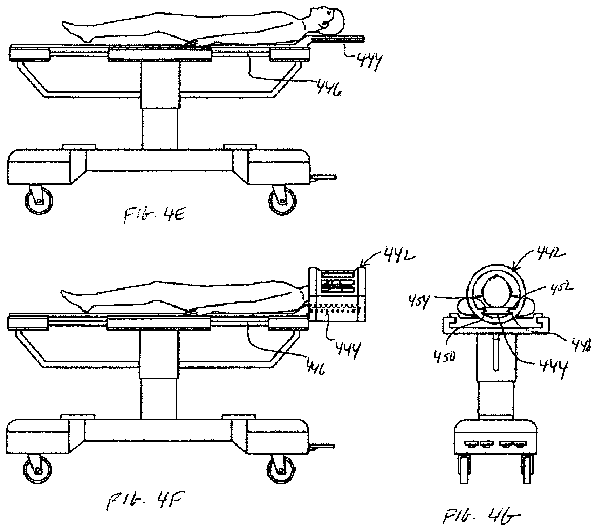

FIG. 4 shows a series of additional patient table configurations that may be used with patient transporter 100 (shown in FIG. 1) in place of the flat imaging configuration that is shown in FIG.1 or the prostate/pelvis patient tables shown in FIG. 2 and FIG. 3. FIG. 4A shows a female patient positioned prone for a breast imaging exam and/or intervention. A dedicated breast imaging and intervention tabletop is shown assembled from the patient support module 182 and a specialized breast anatomical patient support module 410 designed for breast imaging and intervention. The specialized breast anatomical patient module 410 comprises apertures through which the breasts hang pendant. Preferably, these apertures are more than 6" and less than 12" wide in the superior-inferior direction. The specialized breast patient support module 410 may also include receptacles for compression plates (not shown) that hold the breast in a fixed position, grid plates or other guide devices for biopsy needles and other interventional instruments (not shown), and/or receptacles for RF coils (not shown). Electrical connections for RF coils (not shown) may be included in the common table segment 182 and in the specialized breast patient support module 410. Although a specific breast patient support module is described here, the specialized breast patient support module 410 can be constructed to include any of the features described in U.S. Patent US 2005-0080333 and U.S. Patent US 2007-0039101 for their disclosure of breast imaging tabletops, prostate imaging tabletops, and tabletop constructions for imaging other specific anatomical areas.

FIG. 4B shows a male patient positioned supine for lung imaging and/or intervention. A dedicated lung imaging and intervention tabletop is shown assembled from the patient support module 182 and a specialized lung patient support module 420 configured for lung imaging and intervention. The specialized table segment 420 may be constructed from radiolucent material, facilitating a CT examination of the lungs with the patient positioned on the patient table. The specialized lung patient support module 420 may include an aperture under the lungs with a removable cover (not shown) that may be removed for intervention. The specialized lung patient support module 420 may also include guidance devices for performing a lung biopsy, or other interventional procedures. The specialized lung patient support module 420 may include mounting devices for coupling hyperpolarized gas equipment to the patient transporter. FIG. 4C shows a male patient positioned supine for liver imaging and/or intervention. A dedicated liver imaging and intervention tabletop is shown assembled from the patient support module 182 and a specialized liver patient support module 430 designed with an aperture under the liver for liver imaging and intervention. A removable insert 432 may be used to cover the aperture during imaging and removed from the bottom side of the specialized liver patient support module 430 for an interventional procedure without repositioning the patient. The removable insert 432 may comprise RF coil elements for imaging the liver using MRI, or it may comprise an x-ray film cartridge. The specialized liver patient support module 430 may also include guidance devices for performing a liver biopsy, cryotherapy, or other interventional procedures. FIGs. 4D through 4G illustrate a male patient positioned supine for head imaging and/or intervention. A dedicated head imaging and intervention tabletop is shown assembled from the patient support module 182 and a specialized head patient support module 440 designed with a back support portion 446 that ramps up to a head support portion 444. An head RF coil 442 can be coupled to the head support portion, substantially surrounding the head of the patient. As shown here, the head RF coil 442 includes internal flanges 448 and 450 that can be slid into corresponding grooves 452 and 454 formed in the sides of the head support portion 444. Electrical connections for connecting the RF coil 442 to a MRI scanner are preferably included in the patient support module 182 and specialized head patient support module 440. The patient table is shown translated horizontally from the home position such that access to the patient's head from 360 degrees in a vertical plane is provided for performing interventional procedures. For all patient tables, apertures may be covered over during MRI or CT imaging or patient transport, for example. In addition, the patient table may include interventional or therapeutic devices integrated into the table design, for example, focused ultrasound ablation systems or cryoablation systems may be integrated into the table or coupled to the table.

The patient table may be assembled from modules that are completely removable from the patient transporter as shown in FIGS. 1-4. Alternatively, some components of the patient table may be permanently attached to the patient transporter. These permanently attached components may comprise an electrical cabling system for connecting RF coil components to a MRI scanner through the patient table. Electrical connectors on the back end of the patient table may be used to affect an RF connection to an MRI scanner, and there may be multiple electrical connectors along the length of the patient table to allow connection to RF coil elements that may be placed on or coupled to the patient table. The connection of the RF coils to the MRI scanner through the patient table may be effected using direct electrical cabling, or by using fibreoptic connections, wireless or optical connections. Specialized table modules may be coupled to the permanently attached table segments to assemble a full patient bed with dedicated structure and function for facilitating imaging and/or intervention in a particular anatomy.

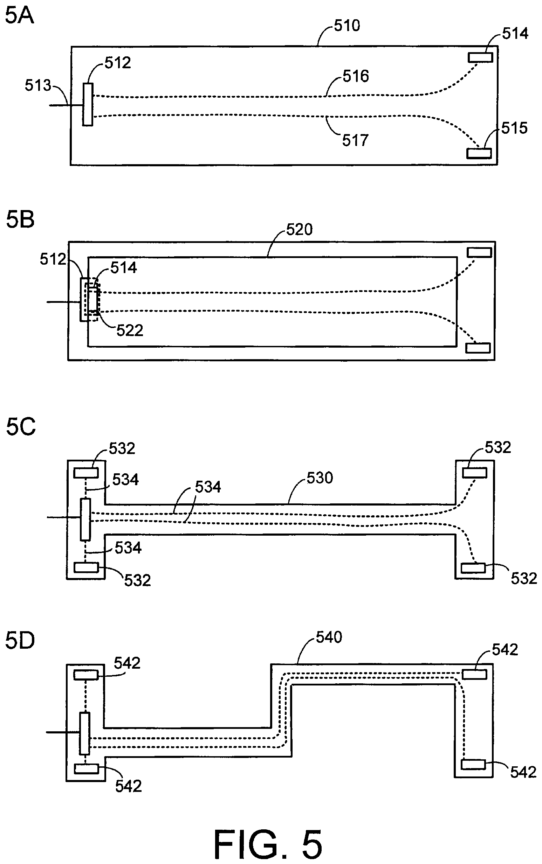

Although the patient support module is described above as connecting horizontally to specialized anatomical patient support modules, the patient support module may also be assembled common backbone structure that receives specialized patient support modules in the top surface. The common backbone structure may accept specialized patient support modules designed specifically for supporting a particular anatomy of interest for MR, CT or PET imaging, for example, a breast, a prostate or other target organ in the pelvis, a liver, a lung, a knee, or a head. These specialized patient support modules are preferably constructed from materials that interfere minimally with imaging techniques such as x-ray, CT, ultrasound, PET and optical imaging. FIG. 5 shows a backbone structure embodiment for a patient table compatible with an MRI scanner comprising electrical cabling and electrical connectors for RF connections. FIG. 5A is a top view of a backbone structure 510, with a scanner connection point 512 for providing an RF connection to an MRI scanner via electrical cabling 513 to the scanner. Connection points for RF coils 514, 515 are located at the front end of the backbone distal to the MRI scanner to allow for convenient attachment of the RF coils to the patient table and obviating the need for having long cables positioned alongside the patient during scanning. The connection points 514, 515 are connected by cabling 516, 517 in the backbone structure 510 to the scanner connection point 512. The backbone structure 510 comprises a bottom layer for assembling a patient table and serves as a foundation for additional specialized structural components. FIG. 5B shows a simple imaging patient table component 520 coupled mechanically to the top of the backbone structure 510 via mating mechanical connectors 514, 522, on the top of the backbone structure 510 and on the patient table 520. While FIG. 5A and 5B shows a single mechanical connection 514, 522, between the backbone structure 510 and the patient table 520, multiple connections may alternatively be used. Alternatively, the patient table 520 may couple mechanically and electrically to the backbone structure 510, and the patient table 520 may additionally comprise connectors for RF coils and electrical connections for providing RF connectivity to the MRI scanner. FIG. 5C-5E show alternate backbone structure embodiments with RF connectivity. The backbone structures shown in FIG. 5C and 5D have been designed to provide increased access to the patient from below the patient table, while still providing RF connections for RF coil elements at the end of the patient table distal to the MRI scanner. FIG. 5C shows an I-shaped backbone structure 530, with RF connections 532 at the proximal and distal ends for connecting RF coil elements. Cabling 534 inside the backbone structure 530 allows for electrical connections between RF coil elements at the distal end of the table to the MRI scanner through the I-shaped backbone structure 530. FIG. 5D shows a backbone structure 540 designed for improved prostate and other pelvic imaging and intervention. RF connections 542 are located at the proximal and distal ends for connecting RF coil elements. This backbone structure 540 provides space for a physician to better access a prostate patient's perineum and/or rectum while performing, interventional procedures.

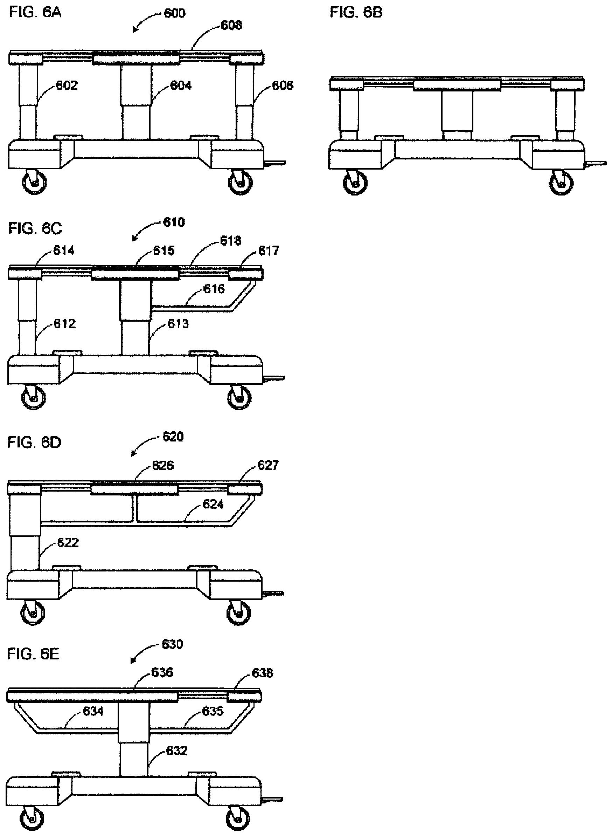

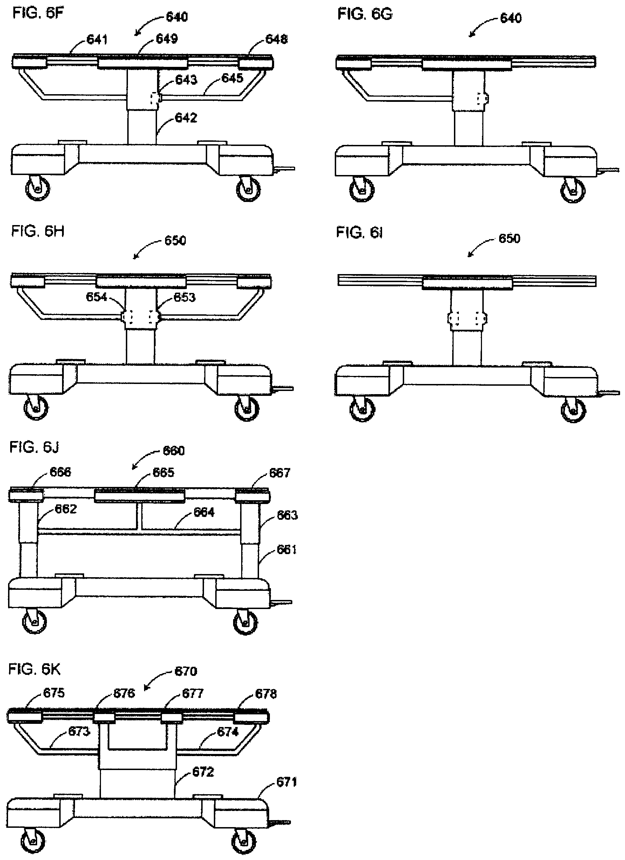

The patient transporter may have a single vertical pedestal as shown in FIGs. 1A - F, or it may have multiple pedestals supporting horizontal platforms. Multiple combinations of pedestals and arms providing cantilevered support to the patient table may be used. The pedestals may be completely separate (i.e., each supported separately on the shared base), or they may be joined together with bridges or other connecting segments. The pedestals may be constrained to move synchronistically to provide vertical adjustment for the patient table, or they may be separately adjustable to provide support at multiple vertical locations for the patient table segments. FIGs. 6A - K show a series of alternate exemplary embodiments for a patient transporter in accordance with the invention. This series of embodiments is not intended to be exhaustive or limiting. FIG. 6A and FIG. 6B show an exemplary patient transporter 600 with three pedestals 602, 604, 606 supporting a patient table 608, with the patient table 608 in the highest and lowest positions respectively. FIG. 6C shows an exemplary patient transporter 610 with two telescoping pedestals 612, 614 and a single arm 616 that supports a cantilevered horizontal platform 617 at the front end of the patient transport. Telescoping pedestals 612 and 613 also support horizontal platforms 614 and 615 respectively. FIG. 6D shows an exemplary patient transporter 620 with a single telescoping pedestal 622 located at one end of the patient transporter 620 supporting an extended arm 624 that supports two cantilevered horizontal platforms 626, 627. FIG. 6E shows an exemplary patient transporter 630 with a central telescoping pedestal 632. Two arms 634, 635 extend horizontally toward the back and front ends of the patient transporter 630 from the central pedestal 632. The back arm 634 supports one end of a long horizontal platform 636 that is also supported at the other end by the central pedestal 632. The front arm 635 supports a second horizontal platform 638. The space between the back horizontal platform 636 and the front horizontal platform 638 provides access to the patient from below the patient bed for ancillary imaging and interventional procedures. FIG. 6F shows an exemplary patient transporter 640 with a central telescoping pedestal 642. Two arms 644, 645 extend horizontally toward the back and front ends of the patient transporter 640 from the central pedestal 642. The front arm 645 is removable from the central pedestal 642, and may be inserted and locked into place on the central pedestal 642 using a locking means 643. FIG. 6G shows the patient transporter 640 with the arm 645 removed. In an alternative embodiment (not shown) the arm 645 may be extendable from within the pedestal 642 by having a telescoping arm extension inside the pedestal 642, such that upon extension of the arm 645, the cantilevered horizontal platform 648 translates horizontally away from a central horizontal platform 649. By removing the arm 645 or retracting the arm 645 into the pedestal 642 when a patient is positioned on a patient table 641, the underside of the patient table 641 may be exposed beneath the patient's head, providing complete access to the patient's head for intervention through apertures in the patient table (not shown). FIG. 6H and 6I show an exemplary patient transporter embodiment 650 with two removable arms. FIG. 6J shows an exemplary transporter embodiment 660 with two pedestals, 662, 663, located one on each end of a wheeled base 661. The pedestals 662, 663 each support a horizontal platform 666, 667, and also provide support to a horizontal arm 664 at each of its ends. The horizontal arm supports a central horizontal platform 665. FIG. 6K shows an exemplary transporter embodiment 670 that comprises a wide central pedestal 672 on a wheeled base 671. The central pedestal 672 supports two horizontal platforms 676, 677 with a large space between them for accessing the patient's torso for interventions. Arms 673, 674 support additional horizontal platforms 675, 678.

The patient transporter may include removable or adjustable structures that provide additional support to the patient table during translation of the patient table into an MRI or CT scanner, for example. These structures may be removed or adjusted to a position remote from the anatomy of interest during an imaging or interventional procedure. For example, insertable or adjustable surfaces may be positioned between the horizontal platforms to create a continuous horizontal surface supporting the patient table during translation. Sliding, rotating, adjustable, foldable, or repositionable surfaces or bridging components may be transposed or repositioned to create a continuous horizontal surface for supporting the patient table.

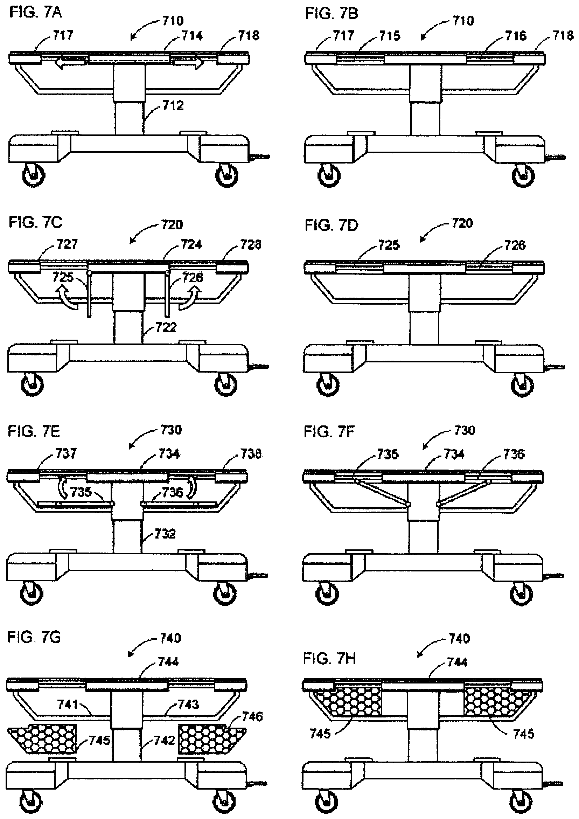

FIGs. 7A - H show a series of exemplary embodiments including reinforcing support structures for supporting the patient table during translation of the patient table. This series of embodiments is not intended to be exhaustive or limiting. FIG. 7A and 7B show an exemplary patient transporter 710 with a central telescoping pedestal 712 that supports a horizontal platform 714. The horizontal platform 714 comprises two sliding support surfaces that may be translated horizontally into the spaces between the horizontal platform 714 and the horizontal platforms 717, 718 as shown in FIG. 7B. FIG. 7C and 7D show an exemplary patient transporter 720 with a central telescoping pedestal 722 that supports a horizontal platform 724. Support surfaces 725, 726 are coupled to the horizontal platform 724 by hinges or other attachment means that allow pivoting from a vertical rest position (shown in FIG. 7C) to a horizontal position (shown in FIG. 7D). Support surfaces 725, 726 are used to fill the spaces between horizontal platform 724 and horizontal platforms 727, 728 to provide support for a patient table during horizontal translation. FIG. 7E and 7F show an exemplary patient transporter 730 with a central telescoping pedestal 732 that supports a horizontal platform 734. Hinged bridge members 735, 736 are attached to the central telescoping pedestal 732 at the front and back ends. These hinged bridge members 735, 736 pivot upward to close off the spaces between central horizontal platform 734 and horizontal platforms 737, 738. FIG. 7G and 7H show an exemplary patient transporter 740 with a central telescoping pedestal 742 that supports a horizontal platform 744. Horizontal arms 741, 742, project horizontally from the central pedestal 746 toward the back and front ends of the patient transporter 740 respectively. Support blocks 745, 746 may be placed on the horizontal arms 741, 742 to provide a continuous horizontal top surface for supporting a patient table during translation. The support blocks 745, 746 may have open mesh sides to allow insertion of devices through the sides.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

## Claims

1. An apparatus for medical imaging and image-guided intervention, the apparatus comprising
- a modular patient table compatible with a medical imaging scanner and
- a transporter to carry the modular patient table, the modular patient table including
- a patient support module and a specialised patient support module removably coupled to the patient support module,
- the patient support module being formed by an I-shaped common backbone structure, and
- the specialised patient support module being configured to support a patient in a position appropriate for imaging a selected anatomy of interest, while also providing an interventional gap under the specific anatomy of interest sized to allow access from below the modular patient table top to the selected anatomy of interest using a medical instrument;
the transporter comprising
- a base
- a central vertical pedestal supported on the base for supporting the patient table top, the vertical pedestal's cross-sectional area being smaller than the modular table's cross-section
- the central vertical pedestal being offset from the interventional gap to provide an open space adjacent the interventional gap to allow access from below the modular patient table top to the selected anatomy using the medical instrument and
- two arms projecting horizontally from the central vertical pedestal towards the back and front ends of the transporter and angle upwards near to ends of the transporter.

2. An apparatus as claimed in Claim 1, wherein the arms are removably mounted to the central vertical pedestal.

3. An apparatus as claimed in Claim 1, wherein the central vertical pedestal supports a central horizontal platform and the arms support additional horizontal platforms, the horizontal platforms comprise engaging tracks for the patient table at their lateral edges.

## Patentansprüche

1. Vorrichtung zur medizinischen Bildgebung und zum bildgeführten Eingriff, wobei die Vorrichtung
- einen modularen Patiententisch, der mit einem Scanner für die medizinische Bildgebung verträglich ist, und
- eine Transportvorrichtung zum Tragen des modularen Patiententisches umfasst, wobei der modulare Patiententisch
- ein Patientenstützmodul und ein Spezialpatientenstützmodul, das mit dem Patientenstützmodul entfernbar gekoppelt ist, umfasst,
- wobei das Patientenstützmodul durch eine 1-förmige gemeinsame Hauptstützstruktur gebildet wird und
- das Spezialpatientenstützmodul so ausgestaltet ist, dass es einen Patienten in einer Position stützt, die für eine Bildgebung einer ausgewählten interessierenden Anatomie geeignet ist, wobei es auch einen Eingriffsabstand unter der spezifischen interessierenden Anatomie bereitstellt, der so bemessen ist, dass er einen Zugang von der Unterseite der modularen Patiententtischplatte zur ausgewählten interessierenden Anatomie mittels eines medizinischen Instruments ermöglicht;
wobei die Transportvorrichtung
- eine Basis
- einen zentralen vertikalen, auf der Basis abgestützten Fuß zur Stützung der Patiententischplatte, wobei die Querschnittsfläche des vertikalen Fußes kleiner als der Querschnitt des modularen Tisches ist
- der zentrale vertikale Fuß vom Eingriffsabstand so versetzt ist, dass ein offener Raum neben dem Eingriffsabstand bereitgestellt wird, um einen Zugang von der Unterseite der modularen Patiententischplatte zur ausgewählten Anatomie mittels des medizinischen Instruments zu ermöglichen, und
- zwei Arme, die vom zentralen vertikalen Fuß in Richtung des hinteren und des vorderen Endes der Transportvorrichtung horizontal hervorragen und nahe den Enden der Transportvorrichtung nach oben abgewinkelt sind, umfasst.

2. Vorrichtung nach Anspruch 1, wobei die Arme am zentralen vertikalen Fuß entfernbar montiert sind.

3. Vorrichtung nach Anspruch 1, wobei der zentrale vertikale Fuß eine zentrale horizontale Plattform stützt und die Arme zusätzliche horizontale Plattformen stützen, wobei die horizontalen Plattformen an ihren seitlichen Rändern Eingriffsschienen für den Patiententisch umfassen.

## Revendications

1. Appareil pour imagerie médicale et intervention guidée par image, l'appareil comprenant
- une table modulaire pour patient compatible avec un scanneur d'imagerie médicale et
- un transporteur pour porter la table modulaire pour patient, la table modulaire pour patient comportant
- un module de support de patient et un module de support de patient spécialisé couplé de manière amovible au module de support de patient,
- le module de support de patient étant formé par une structure d'ossature commune en I, et
- le module de support de patient spécialisé étant configuré pour supporter un patient dans une position appropriée pour imaginer une anatomie d'intérêt sélectionnée, tout en fournissant également un espace d'intervention en dessous de l'anatomie d'intérêt spécifique dimensionné pour permettre l'accès depuis le dessous du plateau de table modulaire pour patient à l'anatomie d'intérêt sélectionnée au moyen d'un instrument médical ;
le transporteur comprenant
- une base
- un socle vertical central supporté sur la base pour supporter le plateau de table pour patient, la surface en section transversale du socle vertical étant plus petite que la section transversale de la table modulaire
- le socle vertical central étant décalé par rapport à l'espace d'intervention pour fournir un espace ouvert adjacent à l'espace d'intervention pour permettre l'accès depuis le dessous du plateau de table modulaire pour patient à l'anatomie sélectionnée au moyen de l'instrument médical et
- deux bras faisant saillie horizontalement à partir du socle vertical central vers les extrémités arrière et avant du transporteur et formant un angle vers le haut à proximité des extrémités du transporteur.

2. Appareil selon la revendication 1, dans lequel les bras sont montés de manière amovible sur le socle vertical central.

3. Appareil selon la revendication 1, dans lequel le socle vertical central supporte une plate-forme horizontale centrale et les bras supportent des plates-formes horizontales supplémentaires, les plates-formes horizontales comprennent des rails de mise en prise pour la table pour patient au niveau de leurs bords latéraux.
